# EUROPEAN PATENT SPECIFICATION

(11) **EP 2 998 387 B1**
(45) Date of publication and mention of the grant of the patent: **03.01.2018**
(21) Application number: 14798394.4
(22) Date of filing: 12.05.2014
(51) Int. Cl.: C12N 1/20, A01N 63/00, C12R 1/25, A01N 63/02

(54) **LACTOBACILLUS PLANTARUM STRAIN FOR THE CONTROL OF FIRE BLIGHT**
LACTOBACILLUS-PLANTARUM-STAMM ZUR BEKÄMPFUNG VON FEUERBRAND
SOUCHE DE LACTOBACILLUS PLANTARIUM POUR LA LUTTE CONTRE LE FEU BACTÉRIEN

(30) Priority: 13.05.2013 ES 201330685
(43) Date of publication of application: 23.03.2016
(73) Proprietor: Universitat de Girona, 17071 Girona (ES)
(72) Inventor: MONTESINOS SEGUÍ, Emilio, E-17820 Banyoles (Gerona) (ES); BONATERRA CARRERAS, Anna, E-17003 Girona (ES); ROSELLÓ PRADOS, Gemma, E-17250 Platja D'Aro (Gerona) (ES); FRANCÉS ORTEGA, Jesús, E-17003 Girona (ES); MONTESINOS BARREDA, Laura, E-17007 Girona (ES); BADOSA ROMAÑO, Esther, E-17811 Santa Pau (Gerona) (ES)
(74) Representative: ZBM Patents - Zea, Barlocci & Markvardsen
(86) International application number: PCT/ES2014/070394
(87) International publication number: WO 2014/184410

(56) References cited:
- US-A1- 2009 169 530
- US-A1- 2009 169 530
- Natalia Viktorivna Limanska ET AL: "Ukrainian Patent Utility Model UA75360U: METHOD FOR PROTECTING PLANTS AGAINST BACTERIAL CANKER USING LACTOBACILLI", Specialized DB "Inventions (Utility Models) in Ukraine", 26 November 2012 (2012-11-26), pages 1-7, XP055298748, Ukraine Retrieved from the Internet: URL:http://base.ukrpatent.org/searchINV/se arch.php?action=viewdetails&IdClaim=181522 &chapter=abstractEN [retrieved on 2016-08-30]
- ROSALIA TRIAS ET AL: "Lactic acid bacteria from fresh fruit and vegetables as biocontrol agents of phytopathogenic bacteria and fungi", INTERNATIONAL MICROBIOLOGY, SPRINGER VERLAG IBERICA, BARCELONA, ES, vol. 11, no. 4, 1 December 2008 (2008-12-01), pages 231-236, XP009165082, ISSN: 1139-6709
- VAN DER ZWET, T. ET AL.: 'Antagonism of lactic acid bacteria against Erwinia amylovora. Phytopathology.' ANNUAL MEETING OF THE POTOMAC DIVISION OFTHE AMERICAN PHYTOPATHOLOGICAL SOCIETY vol. 80, no. 7, 21 March 1990 - 23 March 1990, page 673., XP008182443
- TRIAS, R. ET AL.: 'Bioprotection of Golden Delicious apples and Iceberg lettuce against food borne bacterial pathogens by lactic acid bacteria.' INTERNATIONAL JOURNAL OF FOOD MICROBIOLOGY. vol. 123, no. 1-2, 04 December 2007, pages 50 - 60, XP022511617 DOI: 10.1016/J.IJFOODMICRO.2007.11.065
- HOLT, J.G. ET AL. BERGEY'SMANUAL OF SYSTEMATIC BACTERIOLOGY, 4TH ED. vol. 2, August 1986, pages 1215 - 1216 AND 1229, XP008182484

## Description

The present invention relates to the field of phytosanitary agents, specifically to a new strain of *Lactobacillus plantarum* and its use in the biological control of fire blight in pome fruits and ornamental plants.

### STATE OF THE ART

Between 38 and 42% of production losses of crops worldwide are due to biotic agents (pests, diseases and weeds). To control this problem, conventional agriculture uses pesticides (insecticides, fungicides, bactericides, herbicides), obtained by chemical synthesis. However, the impact of a massive use of synthetic pesticides in the environment and the health of consumers has caused a reorientation of crop protection into an integrated pest management (IPM), which is a strategy that uses a variety of complementary methods: physical, mechanical, chemical, biological, genetic, legal and cultural, to control plant pests. IPM aims to reduce or eliminate the use of synthetic pesticides and to minimize their impact to the environment.

In the field of plant diseases, fire blight of pome fruit trees and related ornamental plants caused by *Erwinia amylovora,* is one of the oldest diseases distributed worldwide that causes great economic losses. The fire blight control is performed by treating the plants with antibiotics (e.g. tetracycline or streptomycin) or copper derivative compounds (Bordeaux mixture, oxychloride, oxide or cooper hydroxide). However, in many countries, particularly in the European Union, the use of antibiotics in agriculture is not allowed. Furthermore, the efficacy of antibiotic treatments in fire blight control is limited by the development of plasmid-borne resistance in *E*. *amylovora.* On the other hand, copper compounds have a negative environmental impact due to their toxicity and accumulation in the environment.

In this sense, the new legislative framework for plant protection products in the European Union covered by Directive 2009/128/EC and Regulation (EC) 107/2009 1 of the European Parliament and of the Council concerning the sustainable use and marketing of pesticides, whose main objective is reducing the risk of the use of pesticides through integrated pest and disease management, and environmental protection, establish that phytosanitary means should be preferably biological and physical.

Therefore it is clear that there is a legislative pressure and a trend towards the use of agricultural means of plant protection not based on synthetic chemical pesticides. Thus, in the new techniques of plant protection the use of biological products is preferred, mainly microorganisms and natural extracts that play an important role. For the control of diseases caused by bacteria, such as fire blight of fruit trees and ornamental plants, the scarce availability of chemicals and limitations for their use has stimulated the development of biological control agents as active ingredients.

Currently there are available at world level some pesticides for biological control of fire blight that include as active ingredients several strains of bacteria such as *Pseudomonas fluorescens* A506, *Pantoea vagans* C9-1, *P. agglomerans* E325, *P. agglomerans* P10c, *Bacillus subtilis* QST713, and *B*. *subtilis* BD170 (Johnson, K. and Stockwell, VO, "Management of fire blight: a case study in microbial ecology" Phytopathol Annu Rev. 1998, vol 36, pp 227-248). In Europe only *Bacillus subtilis* strain QST713 has been authorized so far (Bonaterra A et al, "Prospects and limitations of microbial Biopesticides for Control of bacterial and fungal diseases pomefruit tree" Trees 2012, vol. 26, pp. 215- 226). Also lactic acid bacteria, such as *Lactobacillus plantarum, Lactococcus lactus,* or *Leuconostoc mesenteroides* have been studied as biocontrol agents against phytopathogenic bacteria (Trias R et al. "Lactic acid bacteria from fresh fruit an vegetables as biocontrol agents of phytopathogenic bacteria and fungi" Int. Microbiol. 2008, vol. 11, pp. 231-236).

However, the current technology of fire blight control based on microbial biopesticides is not without problems related to (1) the production of secondary metabolites by most strains, (2) concerns on its biosafety, (3) lack of adequate ecological fitness for colonization of plants, (4) lower efficacy than in antibiotics, and (5) technical difficulties in producing formulated products with long shelf-life comparable to conventional pesticides.

First, various biopesticides described for control of fireblight exhibit and action mechanism consisting in the production of secondary metabolites such as pantocines or cyclolipopeptides. Examples of these biopesticides are *Pantoea vagans* C9-1, *P. agglomerans* E325, *P*. *agglomerans* P10c, *Bacillus subtilis* QST713 and *B*. *subtilis* BD170. However, it has been found that in some cases these secondary metabolites exhibit some toxicity (Ongena M, et al, "Bacillus lipopeptides: versatile biocontrol weapons for plant disease", Trends Microbiol 2008, vol. 16, pp. 115-125). A second aspect is related to biosecurity, which take into account the authorities responsible for the approval of products for human consumption, including plant protection products (EPA in USA, EFSA in Europe), specifically in relation to the possible opportunistic pathogenicity of some bacterial species as *Pseudomonas fluorescens* and *Pantoea agglomerans,* which have been cited as causing clinical infections and sepsis, consideration that can be an obstacle to the authorization of strains of these species for use as a pesticide agent. In the EU, *Pseudomonas fluorescens* strain A506, *Pantoea vagans* C9-1, *P. agglomerans* E325 and *P*. *agglomerans* P10c have not been considered as QPS ("Qualified Presumption of Safety"), a term similar to the known GRAS ("Generally Recognized as Safe"), and therefore are not authorized for biological control of fire blight.

In other cases there is evidence that the ability to colonize and survive in the plant host organs to protect is not efficient, requiring applications at high concentration or complex formulations of these agents to achieve a significant effect of disease control

Furthermore, although they are biological products that have advantages over synthetic pesticides, the fact that they are living organisms implicate that environmental and host conditions may affect its biological activity, making its effectiveness generally variable and significantly lower than that of reference antibiotics.

Finally, the industrial preparation of formulated products involves the processing of microbial cultures obtained by fermentation, to get dehydrated products for long storage. Such processes require drying steps that significantly affect the viability of the microorganisms, limiting the yield of the process, particularly in Gram-negative bacteria such as Pseudomonas and Pantoea, which are more sensitive to thermal and dehydration treatments than Gram-positive bacteria. Given these limitations, it is clear that it is desirable to provide more efficient means for biological control of fire blight of fruit trees and ornamental plants.

### DESCRIPTION OF THE INVENTION

The inventors have isolated a new strain of lactic acid bacteria (LAB) that is very efficient in fire blight control and which overcomes the above limitations. The bacteria of the invention is a strain belonging to the species *Lactobacillus plantarum* that has been isolated from plants from crop fields and natural environments of Mediterranean climate.

Thus, in a first aspect the invention relates to the strain *Lactobacillus plantarum* TC92.

*Lactobacillus plantarum* strain TC92 of the invention, isolated from tomato plants in the region of Girona (Catalonia, Spain) was deposited by the applicant (University of Girona) according to the Budapest Treaty on the 03/13/2013 at the Spanish Type Culture Collection (CECT), located in the Scientific Park of the University of Valencia, Building 3 CUE, Catedrático Augustin Escardino, 9, 46980 Paterna (Valencia), Spain. The strain of *Lactobacillus plantarum* was deposited with the reference TC92 and received the access number CECT 8297 after the International Depositary Authority declared as viable said strain.

The description also discloses mutants of *Lactobacillus plantarum* strain TC92. The term "mutants" refers to derived strains obtained using as starting strain *L*. *plantarum* TC92 of the invention, characterized by maintaining the properties of the deposited strain, as for the antagonistic capacity against phytopathogenic bacteria, biosafety and ecological fitness in the colonization of plants. A "mutant" of *L. plantarum* TC92 is also understood according to the description as a "variant" of *L. plantarum* TC92. An expert in the art will understand that using the strain of the invention as starting material it is routinely possible to obtain, for example by spontaneous mutation or directed mutagenesis, mutants that retain the characteristics and relevant advantages described herein. Methods for obtaining mutants of a given bacterial strain are known in the art. Examples can be found in (Sambrook, J. and Russell, DW "Molecular Cloning: A Laboratory Manual", Chapter 13, "Mutagenesis", Cold Spring Harbor, 3rd Ed., 2001). The fundamental properties that the mutant strains of the disclosure must have are:
(i) antagonist activity against *Erwinia amylovora,*
(ii) presence of genes *pin A, pln B, C pln, pln D, plnJ, plnK, plnM, plnN, plnO, plnP, plnEF* and *plnI* related to the plantaricin synthesis,
(iii) ability to colonize and survive in flowers, leaves and fruits of plants belonging to the *Rosaceae* family, and
(iv) ability to inhibit infections by *E*. *amylovora* in flowers, fruits and leaves of plants in the *Rosaceae* family.

Preferably the mutants have antagonistic activity against at least one other phytopathogenic bacteria such as *Pseudomonas syringae,* and other bacteria such as *E*. *coli, Staphylococcus aureus* and *Bacillus subtilis.*

The presence of plantaricin gene synthesis can be determined by PCR primer pairs defined by the sequences SEQ ID NO: 3-26 as described in Example 1. Antagonist activity can be determined as described in Example 2. The ability to colonize and survive in plants of the family *Rosaceae* may be determined as described in Example 3. The ability to inhibit *E*. *amylovora* infections can be determined as described in Examples 4 and 5.

In the present invention the term "strain of the invention" refers to the strain *L*. *plantarum* TC92.

The strain of the invention has several advantages that make it particularly suitable for use in integrated pest control. In the present invention the term "integrated pest control" (or "integrated pest management") have the usual meaning in the field of agriculture, where it is understood as a strategy that uses a variety of complementary methods: physical, mechanical, chemical, biological, genetic, legal and cultural for pest control. These methods are applied in three stages: prevention, observation and application. It is an ecological methodology that aims to reduce or eliminate the use of synthetic chemical pesticides and to minimize the adverse effects on the environment. It is also said as ecological or biological pest control. Thus, herein the terms "pest control", "biological pest control" are used interchangeably and refer to integrated pest control.

The LAB in general and the species *Lactobacillus plantarum* in particular are safe and suitable for human consumption. Accordingly, the strain of the invention is suitable for use in agriculture. The LAB are considered GRAS by the FDA in the United States and as QPS by EFSA in the European Union.

In addition to its recognized biosafety, the strain of the invention is highly effective in preventing fire blight caused by the bacteria E. amylovora in plants of the *Rosaceae* family. Thus this strain is useful for the biological control of this pest and can be used as pesticide agent. As shown in the Examples below, the effectiveness of the strain of the invention for fire blight control is mainly due to their high antagonist activity against *E*. *amylovora* and its unexpected high ability to colonize and survive in aerial organs of the plants such as leaves, fruits and flowers.

Therefore, in another aspect the invention relates to the use of the strain *Lactobacillus plantarum* TC92 as a plant pesticide.

In the present invention the term "pesticide" means with its usual meaning in the field of agronomy as a product intended to kill, repel, regulate or stop the growth of living organisms considered as pests. Clearly, due to the nature of the strain *Lactobacillus plantarum* TC92, herein, it is understood that "pesticide" is a biological or ecological pesticide, also called biopesticide. In the scope of the present invention the term "pesticide" have the same meaning as the term "phytosanitary agent".

In a particular embodiment of the invention *Lactobacillus plantarum* TC92 is used for control of fireblight in plants of the family *Rosaceae.* In another particular embodiment, the strain of the invention is used in the fire blight control in fruit trees and ornamentals,preferably pear, apple, quince, medlar, hawthorn, rowan, whitebeam (*Sorbus aria*)*,* white hawthorn, snowy mespilus (*Amelanchier ovalis*)*,* hawthorn and laurustinus.

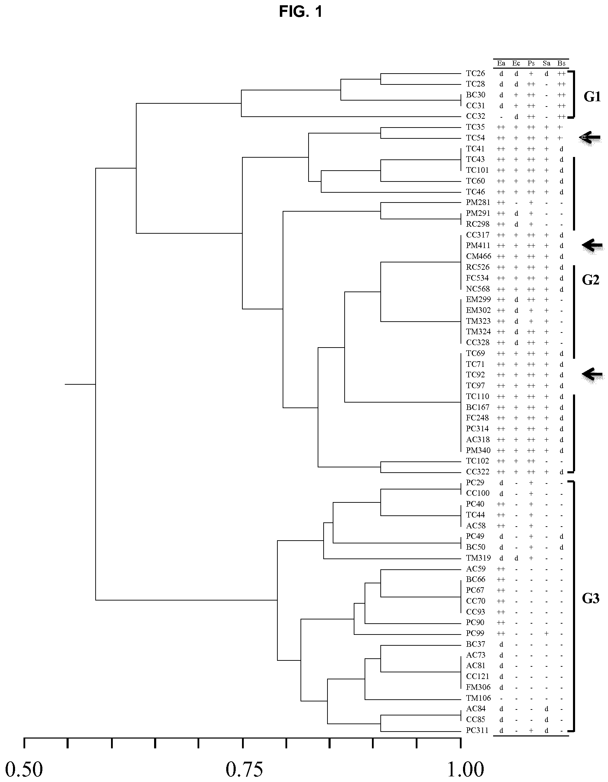

The high antagonistic activity of the strain of the invention is partly due to the production of peptide and non-peptide compounds which inhibit the growth of pathogenic bacteria, including *E*. *amylovora.* One of these compounds is a very active plantaricin encoded by the plnEF gene. Although many strains of *L*. *plantarum* contain the plnEF gene, not all show antagonistic activity against *E*. *amylovora.* In this sense, in Example 1 it shows that many strains of *L. plantarum* plnEF+ (which possess the gene for synthesis of plantaricin EF) have no significant antagonistic activity against *E*. *amylovora* or against other bacteria. In contrast, FIG. 1 shows that the strain of *L.plantarum* TC92 of the invention exhibits an outstanding spectrum of antagonism to *E*. *amylovora* as well as against other bacteria. This unexpected antagonistic effect is also shown in Examples 5 and 6, where the effectiveness of the strain is demonstrated in vivo in inhibiting *E amylovora* infections in plants of the family *Rosaceae.* In Example 1 it is also shown that the strain of the invention has the capacity to produce other plantaricins in addition to the one coded by *plnEF* gene, such as those encoded by the genes *plnJ, plnK, plnM, plnN, PLnO, plnP,* and *plnI.*

Moreover, as demonstrated in Example 3, the strain of the invention has a high ability to colonize and survive on plant organs to protect against fire blight. This amazing ecological fitness is very important for the biological control of the pest.

Facing pesticide use in plants it is important to obtain large amounts of viable cells of the strain of the invention, which is advantageous to have an industrially scalable method to provide concentrated viable cells of easy handling and retaining their viability during storage.

Therefore, another aspect of the invention relates to a process for obtaining cells of the strain of the invention comprising: (i) inoculating the strain of the invention in an appropriate culture medium, (i) subjecting the inoculated culture medium of step (i) to conditions conducive to the growth of the strain of the invention to achieve a cell concentration greater than 1 x 10⁹ CFU / ml, (iii) separating the cells from the culture medium, and (iv) harvesting the cells resulting from step (iii). This procedure is suitable for obtaining the strain of the invention with good viability and maintaining the advantageous properties for use as a pesticide in plants.

The strain of the invention can be inoculated in the growth medium at a final concentration between 1 and 5%. Preferably the inoculated culture is in exponential growth phase. Suitable culture media for the growth of the strain of the invention are synthetic such as MRS (de Man, Rogosa and Sharpe), SL (Lactobacillus selective) and APT (All Purpose medium), media of plant origin like molasses (for example sugarcane, sugarbeet), or of animal origin like dairy subproducts (whey). Suitable growth conditions for the strain are temperatures between 25 and 35 ° C, pH 5 to 7, and oxygen concentration between 15 and 30%. The growth of the strain of the invention preferably occurs under shaking. Cell growth will slow upon reaching, preferably, a cell concentration between 3 x 10⁹ and 8x 10⁹ CFU / ml, more preferably between 4 x 10⁹ and 6 x 10⁹ CFU / ml. An example of the detailed procedure for obtaining cells from the strain of the invention is reflected in Example 7.

In a particular embodiment, the process for obtaining cells of the strain of the invention comprises carrying out a step (v), after step (iv), where cells are subjected to conditions of dehydration. Dehydration can be performed by a lyophilization process. Alternatively, the concentrated material can be dehydrated by fluidized bed drying. Another option is to dehydrate the concentrated material by atomization. In this respect another advantageous characteristic of the strain of the invention is that it exhibits high resistance to dehydration processes which are customary in obtaining microorganisms on an industrial scale. In order to improve the viability of the cells before performing the dehydration process an osmoprotector inert ingredient can be added to a cell concentrate.

In another particular embodiment, the process for obtaining cells of the strain of the invention comprises re-suspending the cells obtained in step (iv) in a suitable buffer to yield a concentrated cell suspension.

With a view to their use in pest control, the pesticide agents are usually formulated into compositions which also include suitable additives for agricultural use for which they are designed. Thus, further aspects of the invention relate to pesticide compositions comprising the strain *Lactobacillus plantarum* TC92, together with agriculturally acceptable compounds and the use of these compositions in the control of fireblight in plants of the family *Rosaceae.* The compositions of the invention may be solid (including, for example, concentrated dehydrated bacteria) or liquid (concentrated bacterial suspensions).

"Suitable compounds for agricultural use" refers to those compounds and/or materials suitable for use in agriculture. Generally, these compounds should be non-toxic to humans and preferably should not have adverse effects to the environment.

In a particular embodiment, the pesticide compositions of the invention may contain compounds for improving adhesion of the strains to the plants being treated, plant-strengthener compounds, nutrients, wetting agents, stabilizers, osmoprotectants, antioxidants, sunscreen protectors, buffering compounds or combinations thereof. Some compounds for improving adhesion are gelatin, starch, pectins, alginates and various types of gums as xanthan. Many of these compounds are also humectants. Among the sunscreen compounds Congo red dyes are included. The plant strengtheners are compounds that can promote crops to develop vigor or tolerance to pathogens or adverse environmental conditions, such analogs of jasmonic acid and certain defense stimulators in plants as harpins, chitosans, and laminarins. In particular, the pesticide compositions, of the invention contain at least one additive as osmoprotector. Examples of osmoprotectant compounds are betains, amino acids and trehalose.

Strategies for physiological improvement of the strain of the invention may also be used to increase the viability of cells of the strain of the invention in the pesticide composition, and subsequent effectiveness in pest control. Such strategies have been described for other biopesticide bacteria (Cabrefiga et al, "Improvement of fitness and efficacy of a fire blight biocontrol agent via nutritional enhancement combined with osmoadaptation. "Applied and Environmental Microbiology, 2011, vol. 77, pp. 3174-3181).

The compositions of the invention may also contain an additional pesticide agent. In one embodiment, the composition of the invention comprises at least one additional pesticide. Such additional pesticide agents may be non-biological, for example, antibiotics or copper derivatives. The advantage of the compositions containing biological pesticides as the strain of the invention in combination with conventional pesticides is that the amount of antibiotic or copper derived coming into contact with the environment is reduced, thus reducing pollution. In addition, conventional pesticide and biopesticides can act in a complementary way to increase the effectiveness of the compositions for pest control. In compositions comprising antibiotics and / or copper compounds it is advantageous to use a mutant of *Lactobacillus plantarum* TC92 resistant to such compounds. Antibiotics may be selected from the group consisting of streptomycin, tetracycline and kasugamycin.

In a particular embodiment, the additional pesticide agent is another bacterial strain effective in fire blight control. Outstanding bacterial strains effective in control of fire blight include, besides the strain of the invention, the *Lactobacillus plantarum* TC54 and *Lactobacillus plantarum* strain PM411. Preferably the composition of the invention comprises *Lactobacillus plantarum* TC92, TC54 Lactobacillus plantarum and *Lactobacillus plantarum* PM411 together with suitable additives for agricultural use.

As with TC92 *Lactobacillus plantarum* strain, the inventors have found that the strains *Lactobacillus plantarum* TC54, as well as mutants thereof, and *Lactobacillus plantarum* PM411, as well as mutants thereof, have advantageous properties for use as pesticides in the biological control of fireblight. These strains are safe, possess high antagonist activity against *E*. *amylovora* and are able to colonize and survive in the plants of the *Rosaceae* family. Thus, the present disclosure also refers to a strain of *Lactobacillus plantarum* TC54 and a strain of *Lactobacillus plantarum* PM411. Other aspects of the disclosure relate to the use of each of these strains as biopesticides in plants, particularly in the control of fireblight in plants of the family *Rosaceae.* Other aspects of the disclosure refer to pesticide compositions comprising at least one of these strains with agriculturally acceptable compounds and the use of these compositions in fire blight control. The disclosure also refers to a process for obtaining cells of *Lactobacillus plantarum* TC54 and / or *Lactobacillus plantarum* PM41, analogous to that described for Lactobacillus plantarum TC92. The description also discloses mutant derivatives of PM411 and TC54 strains. Such mutants must have the above mentioned properties for the TC92 mutant strain.

The strains *Lactobacillus plantarum* TC54 and *Lactobacillus plantarum* PM411 isolated from tomato plants and pear, respectively, from the region of Girona (Catalonia, Spain), were deposited by the applicant (University of Girona) according to the Budapest Treaty 13/03/2013 in Spanish Type Culture Collection (CECT), located in the Scientific Park of the University of Valencia, Building 3 CUE. Profesor Augustin Escardino, 9, 46980 Paterna (Valencia, Spain). The strain of Lactobacillus plantarum TC54 received the access number CECT 8298. The strain of Lactobacillus plantarum PM411 received the accession number CECT 8299. The International Depositary Authority said such strains are viable.

The invention in another aspect relates to an extract of *Lactobacillus plantarum* TC92 strain obtainable by means of a process that comprises: (i) inoculating the *Lactobacillus plantarum* TC92 in appropriate culture medium, (ii) subjecting the inoculated culture medium from step (i) to conditions appropriate for the growth of *Lactobacillus plantarum* TC92, until a cellular concentration above 1 x 10⁹ CFU / ml is achieved, (iii) separating the cells from the culture medium of stage (ii), (iv) collecting cell-free extract, and (v) submit the extract from (iv) to a process of concentration.

Culture media, inoculation conditions and conditions appropriate for growing the strain of the invention to obtain an extract are the same as those defined above for obtaining cells of the bacteria. Preferably, the growth of cells will be slowed down in strationary phase, more specifically after 18-30 hours. In relation to stage (v), non-limiting examples of suitable processes for concentration are dehydration (lyophilization spray-drying), filtration, ultrafiltration, precipitation, centrifugation, and chromatography.

The extracts of Lactobacillus plantarum TC92, obtained according to the procedures provided by the present invention show a strong antagonistic activity against *E*. *amylovora.* In addition, in order to increase its activity, the extracts of the invention can be subjected to a process of fractionation to separate the most active fractions. Preferably, the fractionation comprises a trichloroacetic treatment and/or cation exchange or reverse phase chromatography.

The extracts of the strain *Lactobacillus plantarum* TC92 may be used directly in the biological control of fire blight but, preferably, will be part of a pesticide composition with other agriculturally acceptable compounds. Therefore, further aspects of the invention provide a pesticide composition comprising the extract of the invention and agriculturally acceptable compounds and the use of such compositions for fire blight control.

*Lactobacillus plantarum* TC54 extracts, of *Lactobacillus plantarum* PM411, or compositions comprising them are also useful for fire blight control. These extracts are obtained by a method analogous to that described for obtaining the extract of TC92 strain.

Finally the invention relates to a method of biological control of fire blight in plants of the *Rosaceae* family which comprises treating the plants with the strain of the invention or a composition comprising said strain and/or strain extract of the invention as defined above or a composition comprising said extract.

Throughout the description of the invention and claims the word "comprise" and its variants do not intend to exclude other technical features, additives, components or steps. Also, the word "comprise" includes the case "consists in". For those skilled in the art, other objects, advantages and features of the invention will arise partly from the description and partly from practice of the invention. The following examples and drawings are provided by way of illustration. The numerical symbols relating to the drawings and placed between parentheses in a claim, are only an attempt to increase the understanding of the claim. Furthermore, the present invention covers all possible combinations of particular and preferred embodiments set forth herein.

### BRIEF DESCRIPTION OF THE DRAWINGS

FIG. 1 . Dendrogram corresponding to the dissimilarity coefficient for the 62 strains of *L. plantarum* positive for genes plnEF, according to the in vitro antagonism profile in LBP and MRS agar, against *Erwinia amylovora* (Ea), *Escherichia coli* (**E**c), *Pseudomonas syringae* (Ps), *Staphylococcus aureus* (Sa) and *Bacillus subtilis* (Bs). The symbols of inhibitory activity for each indicator bacteria mean: ++, halus of inhibition greater than 10 mm in both culture media; +, halus of less than 10 mm in both culture media inhibition; d, inhibition halus only in a culture medium; - No halus of inhibition was observed. The three main clusters are indicated at 0.70 dissimilarity level (G1, G2, G3) and the position of the TC92, TC54 and PM411 strains in the dendrogram (arrows) are also indicated.

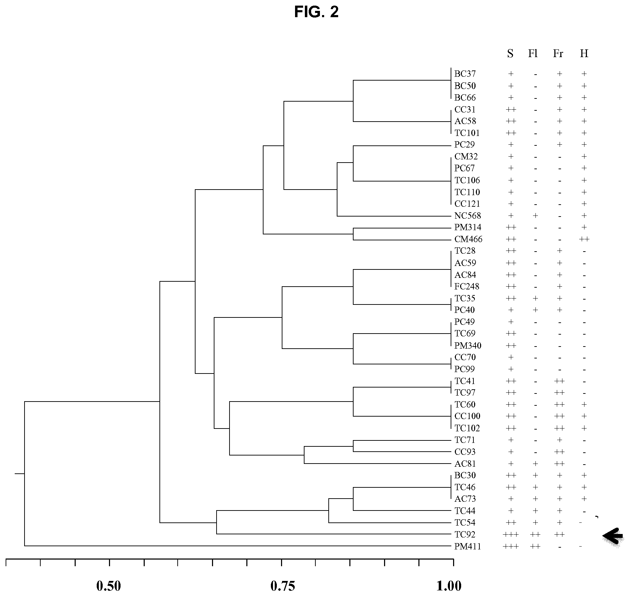

FIG. 2. Dendrogram corresponding to the dissimilarity coefficient for the 41 strains of L. plantarum having inhibitory activity against *Erwinia amylovora,* classified according to the level of inhibition of the culture supernatant (S) and by the ability to protect against fire blight infections in flowers (Fl), fruits (Fr) and leaves (H) of pear tree. In the latter case two experiments were conducted. Symbols indicate in the case of the inhibitory activity of culture supernatants (S): +++, strong, ++ moderate, +, light. Symbols indicate in the case of protection against infections: ++, significant effects in both experiments; +, significant effects in an experiment; -, no effect in any of the experiments. The arrow indicates the position of the TC54, TC92 and PM411 strains.

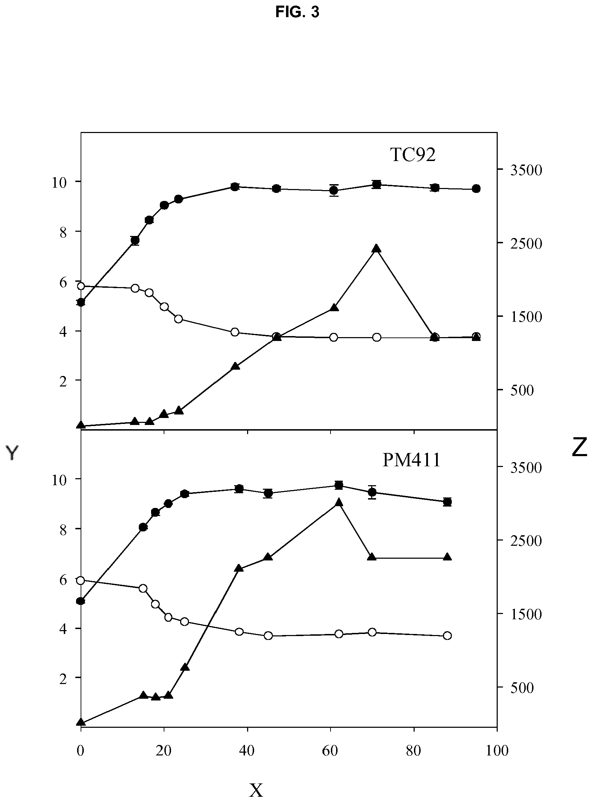

FIG. 3. Kinetics of growth (solid circles) of *Lactobacillus plantarum* strains TC92 and PM411 in MRS broth at 25 °C, and pH evolution (open circles) and antimicrobial activity of the culture supernatant against *Erwinia amylovora* (triangles). X, time (hours). Y, viable cells concentration (Log10 CFU / ml). Z, inhibitory activity (arbitrary units).

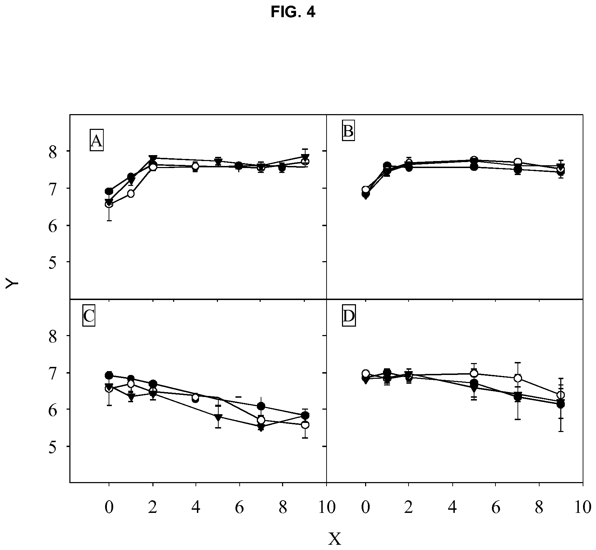

FIG. 4. Growth and survival of *Lactobacillus plantarum* strains TC54 (solid circles), TC92 (open circles) and PM41 1 (triangles) in pear and apple blossoms under conditions of relative humidity (RH), high (90%) or low (50%). A, pear, high RH; B, apple, high RH; C, pear, low RH; D, apple, low RH. X, days after inoculation. Y, viable population level (log10 CFU / flower). The values are the mean of three replicates. Error bars represent 95% confidence interval of the mean.

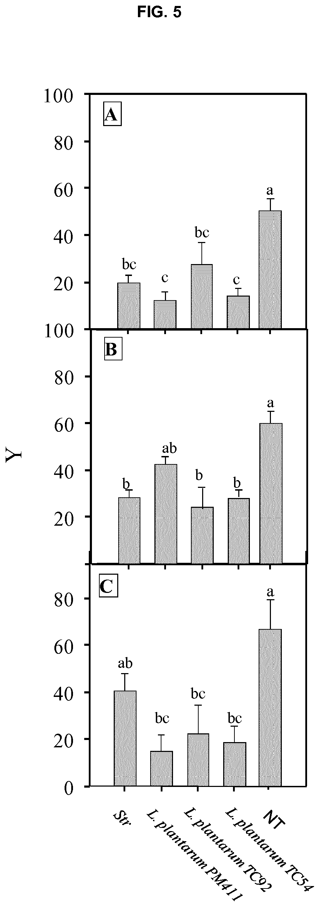

FIG. 5. Effect of the treatments with *Lactobacillus plantarum* strains TC54, TC92 and PM411 in susceptibility to infection by *Erwinia amylovora* in flowers (A), immature fruit (B) and leaves (C) of Passe Crassane pear tree. The intensity of infections compared to an untreated control (NT) and a treatment with the reference antibiotic streptomycin (Str). The values are the mean of three replicates and error bars represent 95% confidence interval of the mean. Same letters on the bars indicate that the treatments did not differ significantly according to Tukey test (P <0.05). Y, intensity of infection (%).

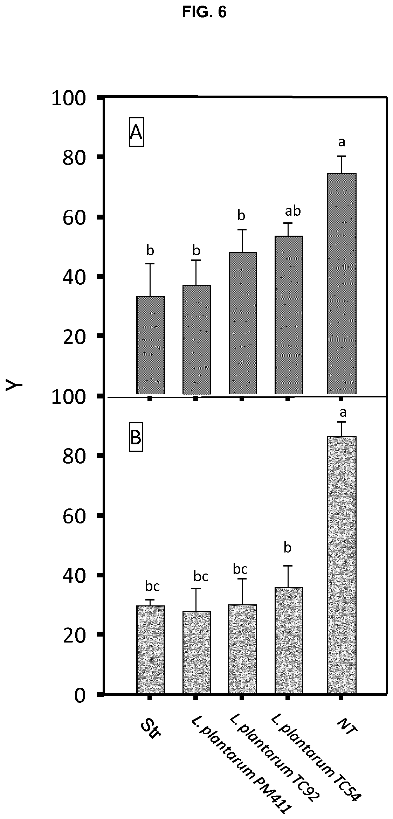

FIG. 6. Effect of treatments with *Lactobacillus plantarum* strains TC54, TC92 and PM411 in the intensity of fireblight infections in plants of Conference pear. The intensity of infection was determined weekly after inoculation of Erwinia amylovora. Two independent experiments (A, B) were performed. The results are compared with an untreated control (NT) and a treatment with the antibiotic streptomycin (Str). The values are the mean of three replicates and error bars represent 95% confidence interval of the mean. Same letters on the bars indicate that the treatments did not differ significantly according to Tukey test (P <0.05). Y, intensity of infection (%).

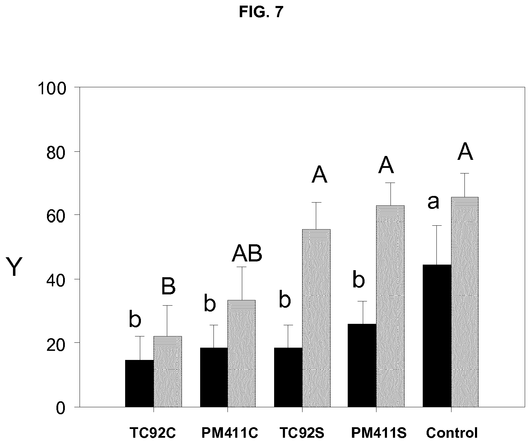

FIGURE 7. Effect of treatments with cells of *Lactobacillus plantarum* PM411C, and TC92C and with diluted 1/10 culture extracts (TC92S and PM411S), in the susceptibility to infections by *Erwinia amylovora* in pear leaves of Conference cultivar. The intensity of infections was compared to an untreated control. The values are the mean of three replicates of 3 leaves, and the error bars represent 95% confidence interval of the mean. Equal letters on the corresponding bars evaluated each day indicate that the treatments did not differ significantly according to Waller test (P <0.05). Y, intensity of infection (%). Gray bars correspond to the levels of infection at 6 days, and black bars at 9 days.

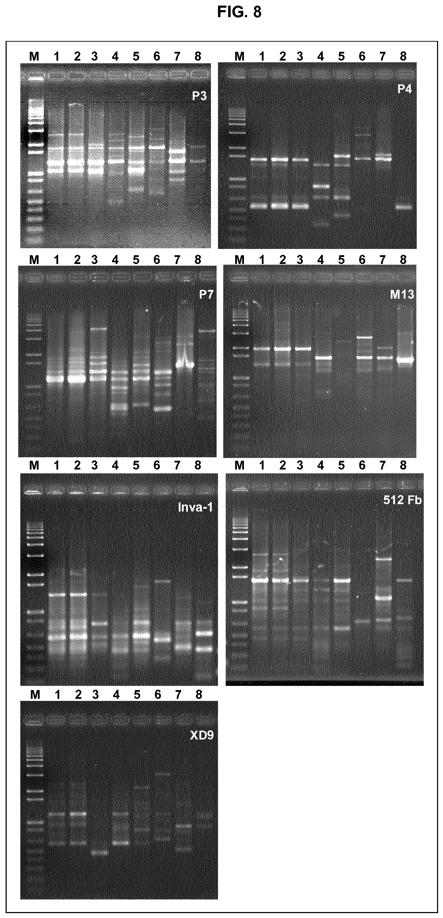

FIGURE 8. Patterns of Random Amplified Polymorphic DNA (RAPD) for the strains of *L. plantarum,* according to different primers: P3, P4, P7, M13, Inv-A, and XD9 512Fb. M, ladder 1 Kb Plus (Invitrogen); 1, *L. plantarum* TC54; 2, *L. plantarum* TC92; 3, *L. plantarum* PM411; 4, *L. plantarum* CECT 223; 5, *L. plantarum* ATCC 8014; 6, *L. plantarum* CECT 4528; 7, *L. plantarum* CECT 5785; 8, *L. plantarum* LNG921 1 (WCSF1).

### EXAMPLES

### Example 1. Isolation and characterization of strains Lactobacillus plantarum TC92, Lactobacillus plantarum TC54 and Lactobacillus plantarum PM41

To isolate the strains samples were taken from leaves, flowers and fruits obtained from commercial orchards of pomefruits and stonefruits, as well as from horticultural products obtained in retail markets and cut products (ready-to-eat salads, sprouts, etc.), all them originally taken from field crops and natural environments under Mediterranean climate. The samples were processed by conventional microbiological methods (Mora et al, "Antimicrobial peptide genes in Bacillus strains from plant environments" International Microbiology, 201 1 vol. 14, pp. 213-223), and lactic acid bacteria (LAB) were isolated in MRS medium at 23 °C for 48 h, consisting finally of a collection of 600 isolates. These isolates were screened in a study to determine the presence of the plnEF gene, responsible for the production of a type of plantaricines (Saenz et al, "Genetic diversity of the pin locus Among oenological Lactobacillus plantarum strains", Int. J. Food Microbiol. 2009, vol. 134, pp. 176-183). DNA from the isolates and from reference strains (*L. plantarum* LMG 921 1 as positive control), and from *Leuconostoc mesenteroides* CM160 (negative control) was used to detect the gene by PCR. The DNA was obtained from exponential phase cultures by extraction in Tris-saline-EDTA-SDS antioxidant buffer and precipitation with isopropanol, adjusting the concentration to 10 ng / microL. The amplification reactions were performed under standard conditions using primers defined by the sequences SEQ ID NO: 25 (*plnEF* forward) and SEQ ID NO: 26 (*plnEF* reverse). The conditions were 4 min at 95 °C, 35 cycles of 1 min at 94 °C, 1 min at 53 °C, and 1 min at 72 °C, with a final extension step of 10 min at 72 °C followed of a stop at 4 °C. Amplified products were separated by agarose gel electrophoresis (1 .5%) in 1X Tris-acetate EDTA (TAE) for 45 min at 90 V, and viewed with SYBR Safe (SYBR®Safe Invitrogen Life Technologies, CA). Among the isolates only 10.5% (63 isolates out of 600) had the *plnEF* marker gene (FIG. 1), indicating a low presence of such genes in the population of isolated LAB from plants.

To determine the species to which the 63 LAB isolates that possessed the genes plnEF belonged, partial sequencing the 16S rDNA (regions V1 to V3) was performed. To do this a PCR was performed on DNA extracted from the cultures with the 27f (SEQ ID NO: 1) and 1492r (SEQ ID NO: 2) primers, under standard conditions (Lane, DJ, "Nucleic acid techniques in bacterial systematics "Stackebrandt, E., and Goodfellow, M., eds., John Wiley and Sons, New York, NY, 1991, pp. 1 15-175). The PCR products were purified, conveniently concentrated and sequenced with a 3730XL sequencer (Macrogen, Seoul, Korea). The sequences obtained were analyzed and aligned by BioEdit Editor Sequencing and homology determined using the BLAST program at the NCBI database data. The sequences of the most representative strains were deposited in the GenBank (*Lactobacillus plantarum* TC92, Ref.N. JX440375 16S; *Lactobacillus plantarum* TC54, Ref. N. EU074830.1, and *Lactobacillus plantarum* PM411, Ref. N. JX440377 16S).

The 62 isolates of L. plantarum having the *plnEF* genes were tested for antimicrobial activity *in vitro* against six indicator bacteria (E. *amylovora* PMV6076, *P. syringae* EPS94, *L. mesenteroides* CM160, *E coli* ATCC5954*,* S. *aureus* ATCC9144 and *B*. *subtilis* EPS2017), by picking colonies in an overlay of lactose-bromocresol purple (LBP) agar, and in modified MRS (MRS0.2). The growth inhibition halus over the indicators were determined after 48 h at 23 °C. The diameter of inhibition hali were normalized and used to perform hierarchical aggregation analysis using the Jaccard dissimilarity coefficient with UPGMA algorithm (NTSYS, Exeter, USA). Three different aggregates were observed depending on the pattern of antibacterial activity at a level of similarity of 0.7, being the most active group G2 (FIG. 1). The remaining two groups had low activity or only against one or two indicator bacteria. Although it might be expected that all strains of *L. plantarum* plnEF+ (possessing the plantaricin EF gene synthesis) present antagonistic activity against *E*. *amylovora* or against other plant pathogenic bacteria, this is not so, since 19 strains are not active or they are only slightly active. Only in group 2 most strains show wide spectrum of antagonism, especially TC54, TC92 PM411 strains. This may be because the said gene is not expressed in some strains or because strains active have additional mechanisms influencing its antagonist capacity against bacteria. It is well known that lactic acid bacteria produce bacteriocins that are very effective against bacteria of the same species and against other Gram positive (and themselves), but rarely against Gram negative bacteria. However, some strains plnEF+ of the invention, contrary to expectations, have a greater antagonism against *E*. *amylovora* and *P*. *syringae* (both are Gram negative bacteria) than against Gram positive (*B. subtilis, S. aureus, L. monocytogenes*)*.* Surprisingly, none of the strains showed activity against *L. mesenteroides* CM160.

In addition, a study of 41 isolates showed antagonistic activity in vitro against *E*. *amylovora* was performed by determining their ability to inhibit pathogen infection on the flowers, immature fruits and leaves, which allowed to further differentiate between plnEF+ isolates. Several experiments on flowers, leaves and fruits of pear of the variety Passe Crassane were made. For this, the plant material was obtained from a commercial orchard near Girona. For assays with flowers once in the laboratory they were placed individually in tubes immersing the pedunculus in a 10% sucrose solution. Immature fruit and leaves were disinfected with diluted hypochlorite solution, washed and excess water was removed. The fruits were injured with a punch (3 wounds per fruit) while in the leaves it was performed a transverse incision in the central nerve. All plant material was inoculated with isolates showing antagonist activity in vitro against *E amylovora,* including strains TC54, TC92 or PM411 at 10*8 CFU/mL (0.4-1 mL per flower, leaf or fruit) using a microsprayer. The material was incubated in clear plastic containers and placed in controlled environment chambers at 25 °C, high relative humidity (RH) and 16-h light-8 h dark. After 24 h the hipanthia of flowers and wounds of fruits or leaves were inoculated with a suspension of *E*. *amylovora* EPS101 at 10*7 CFU / mL. The treated material was incubated under the above conditions for 5 days and the incidence and severity of disease (infection) was determined. The analysis of the resulting dendrogram resulting from the antagonistic capacity and inhibition of infection reveals a remarkable diversity among isolates (FIG. 2). Surprisingly it confirms the unique nature of the TC54, TC92 and PM411 strains that are grouped into aggregates at the bottom of the dendrogram because they show superior efficacy compared to others.

In order to characterize in more detail one of the strains TC92, TC54 and PM411, the presence of plantaricin synthesis related genes, specifically the regulatory operon *plnABCD* (*pln A, pln B, pln C, pln D*) and of production of various plantaricins (*pln K, pln M, pln N, pln O, pln P, pln EF, pln I*) was determined in the strains, and compared to other strains of the collection and deposited in the Spanish Type Culture Collection (CECT), and reference strains described by other authors. This study was performed by growing strains in MRS medium under the conditions described above, extraction of the DNA, and performing PCR using primers specific to the regulatory, transport and synthesis of plantaricins genes. Following are described the sequences of the primers and references describing them.

In the case of pln A gene the conditions and primer pair defined by SEQ ID NO: 3 and SEQ ID NO: 4 as described by Macwana et al. were used ("A bacteriocin PCR array' for 35 identification of bacteriocin-related structural genes in lactic acid bacteria", Journal of Microbiological Methods 2012, vol. 88, pp. 197-204). Other regulatory and synthesis genes were determined by the primer pairs described by Saenz et al., ("Genetic diversity of the pin locus oenological Among Strains Lactobacillus plantarum", International Journal of Food Microbiology, 2009, vol. 134, pp. 176-183), for *plnB* genes (SEQ ID NO: 5 and SEQ ID NO: 6), *plnC* (SEQ ID NO: 7 and SEQ ID NO: 8), *pln D* (SEQ ID NO: 9 and SEQ ID NO: 10), *plnJ* (SEQ ID NO: 1 1 and SEQ ID NO: 12), *pln K* (SEQ ID NO: 13 and SEQ ID NO: 14), *pln M* (SEQ ID NO: 15 and SEQ ID NO: 16), *plnN* (SEQ ID NO: 17 and SEQ ID NO: 18), *pln O* (SEQ ID NO: 19 and SEQ ID NO: 20), *pln P* (SEQ ID NO: 21 and SEQ ID NO: 22), and *pln I* (SEC ID NO: 23 and SEQ ID NO: 24). For gene synthesis of *plnEF* plantaricin primers (SEQ ID NO: 25 and SEQ ID NO: 26) described by Rojo- Bezares et al. were used ("Characterization of a new organization of the plantaricin locus in the inducible bacteriocin-producing Lactobacillus plantarum J23 of grape must origin", Arch Microbiol 2008, vol. 189, pp. 491-499).

As shown in the following table, the TC92, TC54 and PM41 1 strains can be differentiated from other strains by PCR typing of said genes for plantaricins synthesis, giving a positive pattern specific for all of them. In addition the study confirms that the strains of the invention have the potential of producing different types of bacteriocins other than that encoded by the gene *plnEF,* such as those encoded by the genes *plnJ, plnK, plnM, plnN, plnO, plnP* and *pln I.*

Table 1 A and 1 B. Characteristics of *Lactobacillus plantarum* strains TC92, TC54 and PM411 in relation to the presence of genes involved in the synthesis of plantaricins compared with other strains of different origins and reference strains deposited in the Spanish Type Culture Collection (CECT). +, Presence; - Absence; s, PCR amplification of lower intensity than the positive control; nd, not determined.

**Table 1A**

| Strain | Origin | Regulatory operon | | | |
|---|---|---|---|---|---|
| | | *plnA* | *plnB* | *plnC* | *plnD* |
| TC92 | tomato | + | + | + | + |
| TC54 | tomato | + | + | + | + |
| PM411 | pear | + | + | + | + |
| CECT223 | unknown | S | - | s | - |
| ATCC8014 | fermented corn | S | - | - | + |
| CECT4528 | milk | S | - | s | - |
| CECT5785 | cheese | + | + | + | + |
| EC65 | fermented corn | + | + | - | - |
| J51 | wine | + | + | + | + |

**Table 1A**

| Strain | Origin | | | Regulatory operon | | | | | |
|---|---|---|---|---|---|---|---|---|---|
| | | | | *plnA* | *plnB* | *plnC* | | *plnD* | |
| NC8 | silage | | | - | - | - | | + | |

| Table 1B | | | | | | | | | |
|---|---|---|---|---|---|---|---|---|---|
| | | Operons for the synthesis of plantaricins | | | | | | | |
| Strain | Origin | *pln*JKLR | | *pln*MNOP | | | | *pln*EFI | |
| | | *plnJ* | *plnK* | *plnM* | *plnN* | *plnO* | *plnP* | *plnEF* | *plnI* |
| TC92 | tomato | + | + | + | + | + | + | + | + |
| TC54 | tomato | + | + | + | + | + | + | + | + |
| PM411 | pear | + | + | + | + | + | + | + | + |
| CECT223 | unknown | - | - | - | - | - | - | - | - |
| ATCC8014 | corn | - | - | - | - | - | - | + | + |
| CECT4528 | milk | - | - | - | - | - | + | s | + |
| CECT5785 | cheese | - | - | - | - | - | - | + | + |
| EC65 | corn | - | + | nd | + | nd | nd | + | + |
| J51 | wine | - | - | - | - | - | - | + | + |
| NC8 | silage | + | + | - | - | - | + | + | + |

In order to differentiate the TC92, TC54 and PM411 strains from other strains of *L. plantarum* deposited in the Spanish Type Culture Collection (CECT) a random amplified polymorphic DNA analysis was performed, a technique that is known by the acronym RAPD (Random Amplification of Polymorphic DNA), using short arbitrary primers (8-12 nucleotides) that generate after PCR with one primer, amplifications which allow typing of strains (Williams JG et al, "DNA polymorphisms amplified by arbitrary primers are useful as genetic markers. "Nucleic Acids Res., 1190, vol. 18, pp. 6531 -6535). This study was conducted by culturing strains in MRS medium in the conditions described above, DNA extraction, and conducting PCRs with specific primers known in the prior art. The following primers were used: SEQ ID NO: 27 (P3, Tailliez P, et al, "Molecular diversity and relationships With Lactococcus lactis as revelead by rendomly amplified polimorphic DNA (RAPD)" System Appl Microbiol 1998,vol. 21, pp 530-538), SEQ ID NO: 28 (P4), SEQ ID NO: 29 (P7) and SEQ ID NO.: 30 (M13) (Di Cagnio R, et al, "Selection and use of autochtonous mixed starter for lactic acid fermentation of carrots, french beans or marrows, "Int J Food Microbiol, 2008, vol 127, pp 220-228), SEQ ID NO: 31 (Inv-A, K Rahn, et al. "Amplification of an invA gene of Salmonella typhimurium sequence by Polymerase chain reaction as a specific method of detection of Salmonella", Mol Cell Probes, 1992, 6: 271 -279), SEQ ID NO: 32 (512Fb, Holt SM and Cote G L. "Differentiation of dextran-producing strains Leuconostoc modified by a randomly amplified polymorphic DNA protocol", Appl Environ Microbiol, 1998, vol 64, pp 3096-3098), and SEQ ID NO: 33 (XD9, Moschetti GJ, et al "Random amplified polymorphic DNA and amplified ribosomal DNA spacer polymorphism: powerful methods to differentiate Streptococcus thermophilus strains"., Appl. Microbiol., 1998, vol. 85, pp. 25-36). The PCR mix consisted of a total volume of 50 containing 1X buffer µl, 200 µM of dNTPs, 1, 5 mM MgCl2, 0.5 µM primer and 3.75 U of Taq Polymerase (Invitrogen) and 4 µl of DNA (25 ng / µl). The conditions for amplification were primer SEQ ID NO: 30 M13 (i) denaturation at 94 °C for 3 min, (ii) 35 cycles of denaturation at 94 °C for 1 min, 40 °C for 20 s , with a ramp to 72 °C at 0.6 °C/s and (iii) a final extension at 72 °C for 2 min. For primers SEQ ID NO: 27 P3 and SEQ ID NO: 28 P4 conditions were (i) denaturation at 94 °C for 3 min, (ii) 30 cycles of denaturation at 94 °C for 1 min, 36 °C for 2 min and extension at 72 °C for 2 min. For primers SEQ ID NO: 29 (P7), SEQ ID NO: 31 (Inv-A), SEQ ID NO: 32 (512Fb) and SEQ ID NO: 33 (XD9), the conditions were (i) denaturation at 94 °C for 4 min, (ii) 30 cycles of denaturation at 94 °C for 1 min, 35 °C for 1 min, extension at 72 °C for 2 min (iii) a final extension at 72 °C for 5 min. In FIG 8 we can see that the TC92, TC54 and PM41 1 strains can be differentiated from other strains of *L. plantarum* deposited in the Spanish Type Culture Collection (CECT) such as CECT223, ATCC8014, CECT4528, CECT5785 and LMG9211, since their RAPD banding pattern is different with the P3, P4, P7, M13, Inv-A, 512Fb and XD9 primers. These patterns are reproducible in the same experimental conditions for all strains.

The strains TC92 and TC54, although not distinguishable based on their pattern of RAPD, they can be differentiated according to their antagonist activity against different pathogens (see FIG 1) and, in particular, for their inhibitory activity against *Erwinia amylovora* (FIG 2). As shown in the examples below, these strains also showed differences in their ability to colonize and survive in apple and pear flowers (see FIG 4) and their ability to inhibit infection by fire blight (see

FIG 5).

### Example 2. Study of the antagonist activity of the extracts of L. plantarum TC92, L. plantarum TC54 and L. plantarum PM411

The uniqueness of the TC54, TC92 and PM411 strains, highlights the interest in determining the nature of their antagonist and inhibitory activity against *E*. *amylovora.*

First, it was verified that antimicrobial activity existed in the extract resulting from growth in "batch" of such strains in MRS broth (at 30 °C, pH 6.0, 20% pO₂), and that this activity varied throughout the process. For this, aliquots of the cultures were taken at different times, filtered to remove cells and lyophilized, and the resulting material resuspended in citrate buffer. The 15-fold concentrated extract was assayed by the agar overlay technique applying 10 µl aliquots against *E*. *amylovora* inoculated into Mueller-Hinton agar. The antimicrobial activity was determined in arbitrary units (AU) as the inverse of the highest dilution which showed inhibitory activity. While conducting the growth culture curves in "batch", it was also determined the viable cell concentration, pH and the antibacterial activity of the extract as indicated. It is noted that the cultures grow exponentially until 20h after inoculation into fresh medium, subsequently entering the stationary phase, reaching very high cell concentrations of 5 x 10⁹ CFU / mL. The inhibitory activity of the extracts begins with the stationary phase and increases with decreasing pH. However, once it is stabilized at acidic values (pH 3.6-4.0) at 20 h, the antibacterial activity against *E*. *amylovora* continues to increase up to 60-80 h from the start of cultivation. These results are indicative that in addition to acidic compounds that can inhibit *E*. *amylovora,* strains produce other antimicrobial substances (FIG. 3).

The nature of the antibacterial activity of the extracts from cultures was determined also by studying the effect of: (1) by neutralizing the extracts to see if it was related to inhibitory acids, (2) by heat treatment to denature any active proteins, and (3) by protease treatment to confirm their peptide nature. To verify the effect of pH extracts, the pH was adjusted to values of 2, 3, 4, 4.5, 5, 5.5, 6, 7 and 8. For thermal inactivation filtrates were subjected to 50, 70, 90 or 121 °C for 30 min. Susceptibility to proteases was determined by digestion with proteinase K. The assay of inhibitory activity was performed using ELISA microwell system adding the treated crude extracts to cultures of *E*. *amylovora* in LBP broth and by monitoring growth after incubation of the microplates at 25 °C for 72 under stirring. The inhibitory activity was determined by the area under the growth curves and calculated as percentage inhibition compared to untreated controls. The extracts completely retained activity after heat treatment at 120 °C for 10 min and the activity was stable at pH 3-4, but decreased progressively to pH 7.0 and restored again when adjusted to acidic values. The inhibitory activity was completely lost after treatment with proteinase K and decreased dramatically with chymotrypsin. These results confirm that the TC54, TC92 and PM411 strains produce two types of inhibitors of *E*. *amylovora:* acidic compounds and peptidic compounds of thermostable nature.

Further characterization of the extracts of cultures of the TC54, TC92 and PM411 strains was also carried out by means of fractionation. The extracts concentrated by lyophilization were treated with TCA-acetone-DTT (15% TCA, cold acetone, 20 mM DTT) to precipitate peptides and proteins. The resuspended material was applied to reverse phase cation exchange columns, collecting fractions not retained, and eluting the adsorbed compounds with methanol, acetic acid 50% and 100% acetic acid. The fractions obtained were concentrated and lyophilized to remove organic acids used as eluants, and the resulting pellet was dissolved in water. The antibacterial activity of the fractions obtained was assayed by the agar diffusion method, against *E*. *coli* ATCC11775 and *E. amylovora* 6076. It was concluded that a significant portion of activity against both bacteria is due to products of peptidic nature, a group of which are not retained in the chromatographic column, while another is retained and is eluted with methanol.

### Example 3. Colonization of plant organs by strains of L.plantarum for protection against fire blight.

The ability of TC92, TC54 and PM411 strains to colonize and survive in apple and pear flowers was studied. This property is very important to protect the main entrances of infection by *E*. *amylovora* in plants.

Strains TC92, TC54 and PM411 were grown in MRS broth at 30 °C, pH 6.0, p0₂ 20% to a concentration of 5 x 10⁹ CFU / mL (approximately for 20 h). Cells were harvested by centrifugation and resuspended in 1/4 Ringer solution, until a concentration of 1 x 10⁸ CFU / mL. In order to have a system for specific analysis of the strains for the colonization experiment and to avoid interference from native microbiota that may remain after disinfection, spontaneous mutant strains resistant to rifampicin (TC92R and TC54R) and to nalidixic acid (PM411N), were obtained, so that, this property was used as a counter selection tool in viable plate counting by adding the corresponding antibiotics to agar. Flower clusters from trees were taken on a commercial farm near Girona and once in the laboratory were sprayed to runoff point with suspensions of strains at 10⁸ CFU / mL. The flowers are arranged individually with the peduncle immersed in tubes containing a 10% sucrose solution. The material was placed in controlled environment chambers under high (90% RH) or low (50% RH) relative humidity at 25 °C. For each HR experiment 3 replicates of 30 flowers were used. An experiment was conducted with flowers of pear cultivar Passe Crassane and one with Golden apple blossoms. Then samples of 5 flowers were taken at different times (0, 24, 48, 120, 168, and 216 h). The flowers were homogenized in phosphate neutral buffered peptone by a paddle stirrer. The extracts were diluted and the suitable dilutions were plated using an automated spiral plating system onto MRS agar plates, supplemented with the appropriate antibiotics. The plates were incubated at 25 °C for 48h and colonies were counted using an automatic counting system. The population dynamics of the three strains was similar in both apple and pear flowers. Significant differences between population levels were observed depending on the humidity conditions. Under high HR the population levels in all three strains increased 10 times two days after inoculation, keeping population levels close to 10⁸ CFU / flower for 7 days (FIG. 4). In low RH conditions, which are more adverse to bacterial growth, population levels progressively decreased only 10 times in nine days. These results confirm the ability of the strains to colonize and survive in plant organs and protect them against fire blight, especially in flowers that are the most susceptible organs to the disease.

### Example 4 Inhibitory capacity of fire blight infections in plant organs.

To demonstrate the protective capabilities of the TC92, TC54 and PM411 strains, inhibition assays of infections in flowers, immature fruits and leaves of Passe Crassane pear tree were performed.

Strains suspensions at 1 x 10⁸ CFU / mL were obtained similarly as in Example 3. The plant material was obtained from a commercial plantation near Girona, where fire blight had not been detected in the past. For assays with flowers, each individual flower was immersed through the pedunculus in a solution of 10% sucrose in plastic vials. Immature fruits of 6 weeks and young leaves were disinfected by immersion in a dilute sodium hipochlorite and subsequently washed to remove debris. Three wounds per fruit by means of a needle (1 mm) were performed. On leaves a transverse incision in the main nerve was done. Flowers and immature fruits were then sprayed with suspensions of the TC92, TC54 and PM411 strains 10⁸ CFU / mL (0.4-1 ml per flower or fruit) by a microsprayer, while the leaves were inoculated by immersion in the bacterial suspension. Both vials containing the flowers, and the fruits and leaves in racks were incubated in a controlled environment chamber at 25 °C and relative humidity control, 16 h fluorescent light and 8h dark. After 24 h, the hipanthia of flowers, and the wounds of the fruits and leaves were inoculated with 10 µl of a suspension of *E*. *amylovora* EPS101 at 10⁷ CFU / mL. The inoculated material was placed in transparent plastic boxes and incubated for 5 days at the conditions described above. The experimental design consisted of three replicates per treatment with 3 fruits, 10 flowers or 3 leaves per replicate. *L*. *plantarum*-untreated controls as well as controls treated with a reference antibiotic (Streptomycin) at 100 mg / L were included, and two independent experiments were performed. Infection levels in the case of flowers for each flower were determined according to a scale from 0 to 3 depending on the symptoms observed: 0, no symptoms; 1 partial necrosis of hypanthium; 2, Total hypanthium necrosis; 3 necrosis progression through the stem. For fruit each wound was assessed as: 0, no symptoms; 1, presence of wound exudate; 2, presence of exudates and necrosis around the wound; 3 necrosis extending the fruit surface. On leaves the intensity of infections was determined as: 0, no symptoms; 1 presence of wound exudate; 2 presence of exudate and necrosis around the wound; and 3 necrosis progression through the leaf surface. The severity was normalized to the maximum value obtained in the experiment and expressed in relative percentage (0-100).

The inhibitory effect of the 3 strains (TC92, TC54 and PM411) against fire blight was significant compared to untreated controls, and generally did not differ significantly from the reference antibiotic (FIG. 5). The strains TC92 and TC54 differed significantly from the untreated control in all organs studied, while PM411 was only effective in leaves and flowers. Mean efficiencies were between 67 and 78% in leaves and 45-75% in flowers. In the inmature fruits TC92 and TC54 strains reduced infections compared to untreated controls with an average efficiency of 50%. Therefore, this experiment demonstrated the effectiveness of treatment with such fire blight controlling strains in conditions where the pathogen infections are very favored.

### Example 5. Preventative treatment of fire blight in fruit tree plants by L. plantarum strains TC92, TC54 and PM411.

To demonstrate the efficacy of the *L. plantarum* strains of the invention in fire blight control, assays with pear plants of cultivar Conference were performed. The plants were grown in pots of 20 cm diameter. During the winter they were subjected to vernalization and in spring they were pruned to leave 3-4 buds per plant and forced to sprout in the greenhouse. During vegetative growth the plants were fertilized with a solution of 200 ppm N / P / K (20:10:20) and the shoots were 3-4 cm in length with 5-6 young leaves per shoot. For use in assays, a double incision in the midrib of the three leaves expanded in each of the three shoots was performed. Subsequently the plants were sprayed with the corresponding suspension of the strain *L. plantarum* at 10⁸ CFU / mL (obtained similarly as in Example 3) to the runoff point (10 ml per plant). The treated material was covered with plastic bags and maintained at 25 °C for 24 h with a photoperiod of 16h-8h light dark.

Then the wounds previously practiced in the leaves were inoculated with a suspension of *E amylovora* EPS101 at 10⁶ CFU / ml, and the material reintroduced in plastic bags and kept at 25 °C in photoperiod. The experimental design consisted of three repetitions of two plants per treatment. Untreated controls and controls treated with streptomycin at 100 mg / L were also performed. Two independent experiments were done. The intensity of infection was determined according to the same scale described in the previous section for leaves. The three *L. plantarum* strains significantly inhibited bacterial infections in the two fire blight tests conducted in comparison with untreated controls. Disease levels were significantly lower than in the untreated controls for PM411 and TC92 strains in the two experiments (FIG. 6), although only in one of them for the TC54 strain. Efficiency levels were 50-68% for TC92, 35-65% for PM41 1 and 28-58% for TC54.

### Example 6. Effect of extracts of L. plantarum TC92 and L. plantarum PM411 in inhibiting bacterial infections by fire blight on leaves.

To demonstrate the capabilities of fire blight protection of extracts from strains TC92 and PM411, a test of inhibition of infection in pear leaves of Conference pear was done. For each strain a batch culture was prepared as in Example 3. The cells were obtained from a sample taken at 20 h of culture and the concentration was adjusted to 1 x 10⁸ CFU / mL as in Example 3. Extracts of cultures were prepared as indicated in Example 2 from a sample of the culture taken at 70 h in which the antibacterial activity was high. The plant material was obtained from a commercial plantation near Girona, where no fire blight has been detected in the past. Young leaves were disinfected with sodium hypochlorite, then they underwent a transverse incision in the main nerve, and were inoculated with cell suspensions of the TC92 or PM411 strains at 10⁸ CFU / ml by immersion in the cell suspension or extracts of the same strains diluted to 1/10. The leaves were incubated in a controlled environment chamber at 25 °C and relative humidity control, 16 h fluorescent light and 8h dark, and after 24 h they were inoculated with 10 µl of a suspension of *E*. *amylovora* EPS101 at 10⁷ CFU / mL. The inoculated material was incubated in the conditions described above and intensity of infection at 6 and 9 days was determined.

The experimental design consisted of 3 replicates per treatment with 3 leaves per replicate. The intensity of infection was determined as: 0, no symptoms; 1 presence of wound exudate; 2 presence of exudate and necrosis around the wound; and 3 necrosis progression through the leaf surface. The severity was normalized to the maximum value obtained in the experiment and expressed in relative percentage (0-100). It was observed that after 6 days from inoculation of the pathogen both, cells and extracts of the two strains showed lower levels of infection than the untreated control, and did not differ significantly between them (Figure 7). After nine days, TC92 cells maintained their effectiveness while extracts showed less effectiveness. The loss of activity of the extracts over time could be minimized by suitable formulation of the extract, for example by adding suitable compounds for agricultural use that favor conservation of antimicrobial substances or increasing extract concentration.

### Example 7. Industrial scale production and preparation of formulates to extend the shelf-life of L. plantarum TC92.

For industrial scale production strains of the invention can be used at the optimal fermentation conditions described below, using a CSTR type bioreactor in liquid medium, but may also be used based on solid media fermentation techniques. All are standard methods used in the microbiological industry.

To determine the optimal conditions, a Braun Biostat fermenter of 5 liter was used with MRS broth at 30 °C, pH 6.0, 20% p0₂, with ramp, and shaking at 100 rpm. An exponential culture of the strain *L. plantarum* TC92 was inoculated 1 %, and the bioreactor operational parameters were monitored, finishing growth in stationary phase at 12 h, and getting cellular concentrations of 5-8 x 10⁹ CFU/ ml. The culture material was centrifuged at 8000 rpm in a continuous centrifuge SA-1 Westfalia- Separator at 10 l / h and the material collected aseptically. At the end of this stage the biomass was concentrated 10-12 times, resuspended in phosphate buffer to give about 0.5 liters of a concentrate of 8.4 x 10¹⁰ CFU / ml.

Subsequently the concentrate was supplemented with a osmoprotectant inert ingredient (lactose 15%) and dehydrated in a Virtis freeze drier under standard conditions for 36 h, obtaining 40-50 g dry weight (15% active ingredient) with an activity of 2-4 x 10¹¹ CFU / g d.w. Under such conditions a product is obtained with a nearby cell viability of 100%, stable and easy to store and handle, which at the operative dose of 10⁸ CFU / ml allows to prepare 100 liter suspension ready to be applied to the crop plants to be protected. Then the material in sealed under vacuum in bags, and stored at 4 °C, with a shelf-life of more than a year.

## Claims

1. *Lactobacillus plantarum* TC92 strain deposited in the Spanish Type Culture Collection (CECT) with the access number CECT 8297.

2. Use of the strain *Lactobacillus plantarum* TC92 as defined in claim 1, as a pesticide in plants.

3. Use according to claim 2, for the control of fire blight in plants of the *Rosaceae* family.

4. Procedure for obtaining cells of *Lactobacillus plantarum* TC92 strain as defined in claim 1, comprising:
(i) inoculating the *Lactobacillus plantarum* TC92 strain in a culture medium selected from the group consisting of synthetic media, plant origin media, animal origin media, and combinations thereof
(ii) subjecting the inoculated culture medium of step (i) to a temperature between 25 and 35 °C, a pH between 5 and 7, and an oxygen concentration between 15 and 30% until a cell concentration higher than 1 x 10⁹ CFU / ml is achieved,
(iii) separating cells of the *Lactobacillus plantarum* TC92 from the culture medium,
(iv) harvesting the cells of *Lactobacillus plantarum* TC92 derived from step (iii), and optionally,
(v)subjecting the cells obtained in step (iv) to a dehydration process.

5. Composition comprising the strain *Lactobacillus plantarum* TC92 as defined in claim 1.

6. Pesticide composition comprising the strain *Lactobacillus plantarum* TC92 as defined in claim 1 and agriculturally acceptable compounds.

7. Composition of claim 6 wherein the agriculturally acceptable compounds are selected from the group consisting of plant strengtheners, nutrients, wetting agents, compounds that improve adherence, buffering compounds, stabilizers, antioxidants, osmoprotectants and sun protectants.

8. Composition according to any of claims 6-7 comprising at least one additional pesticide.

9. Composition according to claim 8 wherein the additional pesticide is selected from the group consisting in *Lactobacillus plantarum* strain TC54 (deposited in the CECT under access number CECT 8298), *Lactobacillus plantarum* strain PM411 (deposited in the CECT with the access number CECT 8299), an antibiotic and a copper-derived compound.

10. Composition of claim 9 comprising *Lactobacillus plantarum* TC54 and *Lactobacillus plantarum* PM411.

11. Extract from a *Lactobacillus plantarum* strain TC92 as defined in claim 1, said extract being obtainable by the process comprising:
(i) inoculating the *Lactobacillus plantarum* TC92 as defined in claim 1, in an appropriate culture medium,
(ii) subjecting the inoculated culture medium from step (i) to a temperature between 25 and 35 °C, a pH between 5 and 7, and an oxygen concentration between 15 and 30%, until a cell concentration higher than 1 x 10⁹ CFU / ml is achieved,
(iii) separating the cells from the culture medium of step (ii),
(iv) collecting the cell-free extract, and
(v) submit the extract from step (iv) to a concentration process.

12. Composition comprising the extract of *Lactobacillus plantarum* TC92 according to claim 11.

13. Pesticide composition comprising the extract of *Lactobacillus plantarum* TC92 according to claim 11, and agriculturally acceptable compounds .

14. Use of the composition according to any of claims 6-10 or 13 in the control of fireblight in plants of the family *Rosaceae.*

15. Method for biological control of fire blight in plants of the family *Rosaceae* comprising treating said plants with the strain *Lactobacillus plantarum* TC92 according to claim 1, the extract according to claim 11, the composition according to claims 6-10 and / or the composition according to claim 13.

## Patentansprüche

1. *Lactobacillus plantarum* TC92-Stamm, der bei der spanischen Artenkultursammlung (CECT) unter der Hinterlegungsnummer CECT 8297 hinterlegt ist.

2. Verwendung des *Lactobacillus plantarum* TC92-Stamms wie in Anspruch 1 definiert als Pestizid für Pflanzen.

3. Verwendung nach Anspruch 2, zur Kontrolle von Feuerbrand in Pflanzen der Familie der *Rosaceae.*

4. Verfahren zum Erhalten von Zellen des *Lactobacillus plantarum* TC92-Stamms wie in Anspruch 1 definiert, umfassend:
(i) inokulieren des *Lactobacillus plantarum* TC92-Stamms in ein Kulturmedium ausgewählt aus der Gruppe bestehend aus synthetischen Medien, Medien pflanzlichen Ursprungs, Medien tierischen Ursprungs und Kombinationen davon,
(ii) aussetzen des inokulierten Kulturmediums des Schritts (i) einer Temperatur von zwischen 25 und 35 °C, einem pH-Wert zwischen 5 und 7, und einer Sauerstoffkonzentration von zwischen 15 und 30%
bis eine Zellenkonzentration über 1 x 10⁹ CFU / ml erreicht wird,
(iii) trennen der Zellen von *Lactobacillus plantarum* TC92 aus dem Kulturmedium,
(iv) ernten der aus dem Schritt (iii) hergeleiteten Zellen von *Lactobacillus plantarum* TC92 und, wahlweise,
(v) aussetzen der im Schritt (iv) erhaltenen Zellen einem Dehydratationsprozess.

5. Zusammensetzung umfassend den *Lactobacillus plantarum* TC92-Stamm wie in Anspruch 1 beansprucht.

6. Pestizidzusammensetzung umfassend den *Lactobacillus plantarum* TC92-Stamm wie in Anspruch 1 beansprucht und landwirtschaftlich akzeptable Verbindungen.

7. Zusammensetzung des Anspruchs 6, wobei die landwirtschaftlich akzeptablen Verbindungen aus der Gruppe bestehend aus Pflanzenstärkungsmitteln, Nährstoffen, Netzmitteln, Verbindungen, welche die Haftfähigkeit verbessern, Puffermitteln, Stabilisatoren, Antioxidationsmitteln, kompatiblen Soluten und Sonnenschutzmitteln ausgewählt sind.

8. Zusammensetzung nach einem der Ansprüche 6-7 umfassend mindestens ein weiteres Pestizid.

9. Zusammensetzung nach Anspruch 8, wobei das zusätzliche Pestizid ausgewählt aus der Gruppe bestehend aus *Lactobacillus plantarum* TC54-Stamm (hinterlegt bei der CECT unter der Hinterlegungsnummer CECT 8298), *Lactobacillus plantarum* PM411-Stamm (hinterlegt bei der CECT unter der Hinterlegungsnummer CECT 8299), einem Antibiotikum und einer aus Kupfer basierten Verbindung.

10. Zusammensetzung des Anspruchs 9 umfassend *Lactobacillus plantarum* TC54 und *Lactobacillus plantarum* PM411.

11. Extrakt aus einem *Lactobacillus plantarum* TC92-Stamm wie in Anspruch 1 definiert, wobei der Extrakt durch das Verfahren erhalten werden kann, welches folgendes umfasst:
(i) inokulieren des *Lactobacillus plantarum* TC92 wie in Anspruch 1 definiert, in ein geeignetes Kulturmedium,
(ii) aussetzen des inokulierten Kulturmediums aus dem Schritt (i) einer Temperatur von zwischen 25 und 35 °C, einem pH-Wert zwischen 5 und 7, und einer Sauerstoffkonzentration von zwischen 15 und 30%, bis eine Zellenkonzentration über 1 x 10⁹ CFU / ml erreicht wird,
(iii) trennen der Zellen aus dem Kulturmedium des Schritts (ii),
(iv) auffangen des zellenfreien Extrakts, und
(v) aussetzen des Extrakts aus dem Schritt (iv) einem Konzentrierungsverfahren.

12. Zusammensetzung umfassend den Extrakt von *Lactobacillus plantarum* TC92 nach Anspruch 11.

13. Pestizidzusammensetzung umfassend den Extrakt von *Lactobacillus plantarum* TC92 nach Anspruch 11 und landwirtschaftlich akzeptable Verbindungen.

14. Verwendung der Zusammensetzung nach einem der Ansprüche 6-10 oder 13 zur Kontrolle von Feuerbrand in Pflanzen der Familie der *Rosaceae.*

15. Verfahren zur biologischen Bekämpfung von Feuerbrand in Pflanzen der Familie der *Rosaceae* umfassend das Behandeln der Pflanzen mit dem *Lactobacillus plantarum* TC92-Stamm nach Anspruch 1, dem Extrakt nach Anspruch 11, der Zusammensetzung nach den Ansprüchen 6-10 und / oder der Zusammensetzung nach Anspruch 13.

## Revendications

1. Souche de *Lactobacillus plantarum* TC92 déposée dans la collection espagnole de cultures type (CECT) sous le numéro de dépôt CECT 8297.

2. Utilisation de la souche de *Lactobacillus plantarum* TC92 telle que définie dans la revendication 1, comme pesticide pour plants.

3. Utilisation selon la revendication 2, pour la lutte contre le feu bactérien dans des plants de la famille des *Rosaceae.*

4. Procédé d'obtention de cellules de la souche de *Lactobacillus plantarum* TC92 telle que définie dans la revendication 1, comprenant :
(i) inoculer la souche de *Lactobacillus plantarum* TC92 dans un milieu de culture choisi dans le groupe constitué de milieux synthétiques, milieux d'origine végétale, milieux d'origine animal, et des combinaisons de ceux-ci
(ii) soumettre le milieu de culture inoculé de l'étape (i) à une température d'entre 25 et 35 °C, un pH d'entre 5 et 7, et une concentration d'oxygène d'entre 15 et 30 % jusqu'à atteindre une concentration cellulaire supérieure à 1 x 10⁹ CFU / ml,
(iii) séparer des cellules de *Lactobacillus plantarum* TC92 du milieu de culture,
(iv) récolter les cellules de *Lactobacillus plantarum* TC92 dérivées de l'étape (iii) et, facultativement,
(v) soumettre les cellules obtenues dans l'étape (iv) à un processus de déshydratation.

5. Composition comprenant la souche de *Lactobacillus plantarum* TC92 telle que définie dans la revendication 1.

6. Composition de pesticide comprenant la souche de *Lactobacillus plantarum* TC92 telle que définie dans la revendication 1 et des composés acceptables en agriculture.

7. Composition de la revendication 6 dans laquelle les composés acceptables en agriculture sont choisis dans le groupe constitué de matériaux fortifiants de plantes, nutriments, agents mouillants, composés qui améliorent l'adhérence, composés tampons, stabilisateurs, antioxydants, osmoprotecteurs et protecteurs solaires.

8. Composition selon l'une quelconque des revendications 6-7 comprenant au moins un pesticide additionnel.

9. Composition selon la revendication 8 dans laquelle le pesticide additionnel est choisi dans le groupe constitué de la souche de *Lactobacillus plantarum* TC54 (déposée dans le CECT sous le numéro de dépôt CECT 8298), la souche de *Lactobacillus plantarum* PM411 (déposée dans le CECT sous le numéro de dépôt CECT 8299), un antibiotique et un composé dérivé du cuivre.

10. Composition de la revendication 9 comprenant du *Lactobacillus plantarum* TC54 et du *Lactobacillus plantarum* PM411.

11. Extrait d'une souche de *Lactobacillus plantarum* TC92 telle que définie dans la revendication 1, ledit extrait pouvant être obtenu par le procédé comprenant :
(i) inoculer la *Lactobacillus plantarum* TC92 telle que définie dans la revendication 1, dans un milieu de culture approprié,
(ii) soumettre le moyen de culture inoculé de l'étape (i) à une température d'entre 25 et 35 °C, un pH d'entre 5 et 7 et une concentration d'oxygène d'entre 15 et 30 %, jusqu'à atteindre une concentration cellulaire supérieure à 1 x 10⁹ CFU / ml,
(iii) séparer les cellules du moyen de culture de l'étape (ii),
(iv) recueillir l'extrait exempt de cellules, et
(v) soumettre l'extrait de l'étape (iv) à un procédé de concentration.

12. Composition comprenant l'extrait de *Lactobacillus plantarum* TC92 selon la revendication 11.

13. Composition de pesticide comprenant l'extrait de *Lactobacillus plantarum* TC92 selon la revendication 11, et des composés acceptables en agriculture.

14. Utilisation de la composition selon l'une quelconque des revendications 6-10 ou 13 dans la lutte contre le feu bactérien en plants de la famille des *Rosaceae.*

15. Procédé de lutte contre le feu bactérien en plants de la famille des *Rosaceae* comprenant traiter lesdites plants avec la souche de *Lactobacillus plantarum* TC92 selon la revendication 1, l'extrait selon la revendication 11, la composition selon les revendications 6-10 et / ou la composition selon la revendication 13.


### SEQUENCE LISTING

<110> Universitat de Girona
<120> Lactobacillus plantarum strain for the control of fire blight
<130> P2598ES00
<160> 33
<170> PatentIn version 3.5
<210> 1
   <211> 20
   <212> DNA
   <213> artificial sequence
<220>
   <223> primer 27f
<400> 1
   agagtttgat cmtggctcag 20
<210> 2
   <211> 15
   <212> DNA
   <213> artificial sequence
<220>
   <223> primer 1492r
<400> 2
   accttgttac gactt 15
<210> 3
   <211> 27
   <212> DNA
   <213> artificial sequence
<220>
   <223> primer pknA f
<400> 3
   agcaacttag taataaggaa atgcaaa 27
<210> 4
   <211> 27
   <212> DNA
   <213> artificial sequence
<220>
   <223> primer plnA r
<400> 4
   acagtttctt tacctgttta attgcag 27
<210> 5
   <211> 27
   <212> DNA
   <213> artificial sequence
<220>
   <223> primer plnB f
<400> 5
   gcttcttatt taagtagagg atttctg 27
<210> 6
   <211> 20
   <212> DNA
   <213> artificial sequence
<220>
   <223> primer plnB r
<400> 6
   gccacgatta ctacccttag 20
<210> 7
   <211> 19
   <212> DNA
   <213> artificial sequence
<220>
   <223> primer plnC f
<400> 7
   agcagatgaa attcggcag 19
<210> 8
   <211> 20
   <212> DNA
   <213> artificial sequence
<220>
   <223> primer plnC r
<400> 8
   ataatccaac ggtgcaatcc 20
<210> 9
   <211> 20
   <212> DNA
   <213> artificial sequence
<220>
   <223> primer plnD f
<400> 9
   tgaggacaaa cagactggac 20
<210> 10
   <211> 22
   <212> DNA
   <213> artificial sequence
<220>
   <223> primer plnD r
<400> 10
   gcatcggaaa aattgcggat ac 22
<210> 11
   <211> 18
   <212> DNA
   <213> artificial sequence
<220>
   <223> primer plnJ f
<400> 11
   taacgacgga ttgctctg 18
<210> 12
   <211> 24
   <212> DNA
   <213> artificial sequence
<220>
   <223> primer plnJ r
<400> 12
   aatcaaggaa ttatcacatt agtc 24
<210> 13
   <211> 22
   <212> DNA
   <213> artificial sequence
<220>
   <223> primer plnK f
<400> 13
   ctgtaagcat tgctaaccaa tc 22
<210> 14
   <211> 18
   <212> DNA
   <213> artificial sequence
<220>
   <223> primer plnK r
<400> 14
   actgctgacg ctgaaaag 18
<210> 15
   <211> 24
   <212> DNA
   <213> artificial sequence
<220>
   <223> primer plnM f
<400> 15
   aagcggtata ttaaaagcgt agag 24
<210> 16
   <211> 24
   <212> DNA
   <213> artificial sequence
<220>
   <223> primer plnM r
<400> 16
   catttcctcc ttaaagcatt caac 24
<210> 17
   <211> 19
   <212> DNA
   <213> artificial sequence
<220>
   <223> primer plnN f
<400> 17
   attgccgggt taggtatcg 19
<210> 18
   <211> 19
   <212> DNA
   <213> artificial sequence
<220>
   <223> primer plnN r
<400> 18
   cctaaaccat gccatgcac 19
<210> 19
   <211> 24
   <212> DNA
   <213> artificial sequence
<220>
   <223> primer plnO f
<400> 19
   tggattaaca aagggagaat atgc 24
<210> 20
   <211> 19
   <212> DNA
   <213> artificial sequence
<220>
   <223> primer plnO r
<400> 20
   tcggacccct ctgattcac 19
<210> 21
   <211> 23
   <212> DNA
   <213> artificial sequence
<220>
   <223> primer plnP f
<400> 21
   aaagtatgga caaatgacag tgg 23
<210> 22
   <211> 24
   <212> DNA
   <213> artificial sequence
<220>
   <223> primer plnP r
<400> 22
   aatataaagt tccccaaagc agac 24
<210> 23
   <211> 26
   <212> DNA
   <213> artificial sequence
<220>
   <223> primer plnI f
<400> 23
   gatactcaat aacttccaat gcttag 26
<210> 24
   <211> 24
   <212> DNA
   <213> artificial sequence
<220>
   <223> primer plnI r
<400> 24
   ttgtagcgat aactgaagaa tacg 24
<210> 25
   <211> 21
   <212> DNA
   <213> artificial sequence
<220>
   <223> primer plnEF f
<400> 25
   ggcatagtta aaattccccc c 21
<210> 26
   <211> 19
   <212> DNA
   <213> artificial sequence
<220>
   <223> primer plnEF r
<400> 26
   caggttgccg caaaaaaag 19
<210> 27
   <211> 10
   <212> DNA
   <213> artificial sequence
<220>
   <223> primer P3
<400> 27
   ctgctgggac 10
<210> 28
   <211> 10
   <212> DNA
   <213> artificial sequence
<220>
   <223> primer P4
<400> 28
   ccgcagcgtt 10
<210> 29
   <211> 10
   <212> DNA
   <213> artificial sequence
<220>
   <223> primer P7
<400> 29
   agcagcgtgg 10
<210> 30
   <211> 15
   <212> DNA
   <213> artificial sequence
<220>
   <223> primer M13
<400> 30
   gagggtggcg gttct 15
<210> 31
   <211> 26
   <212> DNA
   <213> artificial sequence
<220>
   <223> primer Inv-A
<400> 31
   gtgaaattat cgccacgttc gggcaa 26
<210> 32
   <211> 26
   <212> DNA
   <213> artificial sequence
<220>
   <223> primer 512Fb
<400> 32
   gtgaaattat cgccacgttc gggcaa 26
<210> 33
   <211> 10
   <212> DNA
   <213> artificial sequence
<220>
   <223> primer XD9
<400> 33
   gaagtcgtcc 10